Europäisches Patentamt

European Patent Office

Office européen des brevets

(11) Publication number: **0 501 375 A1**

# EUROPEAN PATENT APPLICATION

(21) Application number: **92103059.9**

(22) Date of filing: **24.02.92**

(51) Int. Cl.⁵: **C12N 9/96**

(30) Priority: **28.02.91 JP 34400/91**

(43) Date of publication of application:
**02.09.92 Bulletin 92/36**

(84) Designated Contracting States:
**DE DK FR GB NL**

(71) Applicant: **KAO CORPORATION**
**14-10, Nihonbashi Kayabacho 1-chome**
**Chuo-ku Tokyo(JP)**

(72) Inventor: **Yamada, Naoto**
**8762-23, Doaihoncho 1-chome, Hasaki-machi**
**Kashima-gun, Ibaraki(JP)**
Inventor: **Shoga, Yutaka**
**8762-23, Doaihoncho 1-chome, Hasaki-machi**
**Kashima-gun, Ibaraki(JP)**

(74) Representative: **Hansen, Bernd, Dr.**
**Dipl.-Chem. et al**
**Hoffmann, Eitle & Partner Patent- und**
**Rechtsanwälte Arabellastrasse 4 Postfach**
**81 04 20**
**W-8000 München 81(DE)**

(54) Solid enzyme preparation and process for producing the same.

(57) A solid enzyme preparation comprising an enzyme and a disaccharide and a process for the production of a solid enzyme preparation in which an aqueous enzyme solution containing a disaccharide is solidified by means of spray drying are disclosed. The solid enzyme preparation has excellent thermal stability and is not deactivated by heat treatment even after a prolonged period of treating time. The solid enzyme preparation also has excellent resistance to mechanical pressure. In addition, according to the present invention, enzymes are hardly deactivated during spray drying treatment, thereby a solid enzyme preparation of high specific activity can be produced.

FIELD OF THE INVENTION

This invention relates to a solid enzyme preparation and a process for producing the same.

BACKGROUND OF THE INVENTION

Recently, enzymes have been used not only in pharmaceutical preparations and food articles but also in various other industrial fields such as of fibers, leathers, detergents and the like. In general, enzymes have a disadvantage in that they are apt to receive deactivation due to heat-induced structural changes or, in the case of proteolytic enzymes, due to decomposition by themselves. It is therefore desirable to distribute and use enzyme preparations in the form of solid preparations such as powders, granules and the like rather than liquid forms.

Such solid enzyme preparations are conventionally produced by means of freeze-drying or spray drying. Since freeze-drying process is unsuitable for the purpose of a large scale production, spray drying is being used as the most fitted process for the industrial mass production of solid enzyme preparations. Especially, in the case of solid enzyme preparations to be used in detergents, spray drying process is most frequently used because the preparations are required to be produced in the form of granules in view of stability of the preparation and safety for workers and users on inhalation of the preparation.

When a solid enzyme preparation is manufactured in spray drying process, an enzyme solution is generally exposed to a high temperature of 50°C or more, thus the activity of the resulting solid enzyme preparation decreases. In order to improve stability of enzyme preparations under such a high temperature, a process in which sorbitol is used was proposed in U.S. Patent 3,515,642. However, this process merely attains to provide solid enzyme preparations stably at a temperature of 40°C at most. Thus, any satisfactory processes by which a solid enzyme preparation is provided stably even under a spray drying condition of at a temperature of 50°C or more have not been proposed.

SUMMARY OF THE INVENTION

Therefore, it is an object of the present invention to provide a solid enzyme preparation having high thermal stability and a process for producing a solid enzyme preparation using spray drying without causing decrease in the enzyme activity.

The present inventors have conducted intensive studies with the aim of attaining the above object and found that an aqueous enzyme solution hardly cause decrease in an enzyme activity during usual spray drying treatment when a disaccharide is present in the aqueous solution, and that the resulting solid enzyme preparation containing the disaccharide is excellent in thermal stability.

Accordingly, the present invention provides a solid enzyme preparation comprising an enzyme and a disaccharide.

The present invention also provides a process for producing a solid enzyme preparation which comprises solidifying an aqueous enzyme solution containing a disaccharide by spray drying.

Other objects and advantages will be made apparent as the description progresses.

DETAILED DESCRIPTION OF THE INVENTION

Although the disaccharide to be used in the present invention is not particularly limited, it includes, for example, a maltose type disaccharide, a trehalose type disaccharide and a mixture thereof. Examples of the maltose type disaccharide include maltose, cellobiose, gentiobiose, melibiose, lactose, turanose, sophorose and the like; and examples of the trehalose type disaccharide include trehalose, isotrehalose, sucrose, isosucrose and the like. Among them, the maltose type disaccharide is preferred and lactose is more preferred.

Although an amount of the disaccharide to be included in a solid enzyme preparation may vary depending on the type of the enzyme used, it is preferably from 1 to 100 % by weight, more preferably from 10 to 100 % by weight, based on the weight of the enzyme.

Although an enzyme to be used in the present invention is not particularly limited, it includes, for example, a lipase, a cellulase, a protease, an amylase, an esterases, an dextranase and the like. These enzymes are used either alone or a combination of two or more of them. Among these enzymes, cellulases and proteases are preferred, with an alkaline protease K-16 being most particularly preferred.

The alkaline protease K-16 is an enzyme produced by Bacillus sp. KSM-K16 (Fermentation Research Institute Deposition Number FERM P-11418, International Deposition Number FERM BP-3376 under the Budapest Treaty; deposited in Fermentation Research Institute, Agency of Industrial Science Technology, Japan, whose full address of 1-3, Higashi 1-chome, Tsukuba-shi, Ibaraki 305 Japan). Bacteriological properties of the strain KSM-16 are as follows. Bacteriological properties:

(A) Morphological characteristics:

(a) Shape and size of cells: rod of 0.8-1.0 $\mu$m x 2.2-25 $\mu$m;
(b) Pleomorphicity: negative;
(c) Motility: motile and have peritrichous flagella;
(d) Spore (size, shape and location): 1.0-1.2 $\mu$m x 1.4-2.2 $\mu$m, oval shaped, subterminal, slightly swollen sporangia;
(e) Gram stain: positive;
(f) Acid fast: negative;
(g) Colonies grown on bouillon agar plate medium: circular, leafy, smooth surface, pale yellow and translucent;
(h) Colonies grown on bouillon agar slant medium: irregular leafy, slightly rough smooth surface, pale yellow and translucent;
(i) Bouillon broth culture: good growth, turbid, no pellicle formation;
(j) Bouillon-gelatin stab culture: good growth, no gelatin liquefaction;
(k) Litmus milk: peptonization without coagulation of milk, no changes in litmus color;

(B) Physiological characteristics:

(a) Nitrate reduction: positive;
(b) Denitrification: negative;
(c) MR test: negative;
(d) VP test: positive;
(e) Formation of indole: negative;
(f) Formation of hydrogen sulfide: negative;
(g) Hydrolysis of starch: positive;
(h) Utilization of citric acid: positive;
(i) Utilization of inorganic nitrogen source: utilizes nitrates but not ammonium salts;
(j) Formation of pigments: negative;
(k) Urease test: negative;
(l) Oxidase test: positive;
(m) Catalase test: positive;
(n) Temperature for growth: not more than 55°C;
(o) pH for growth: pH 6.6-10.3;
(p) Relation to oxygen: aerobic;
(q) OF test: oxidation type (O type);
(r) Resistance to sodium chloride: grows in the presence of 10% sodium chloride;
(s) Formation of acid and gas from sugars: shown in Table 1;

TABLE 1

| Sugars | Acid formation |
|---|---|
| D-ribose | + |
| L-arabinose | + |
| D-xylose | + |
| D-fructose | + |
| D-glucose | + |
| D-mannose | + |
| D-galactose | + |
| Maltose | + |
| Sucrose | + |
| Lactose | + |
| Trehalose | + |
| Starch | + |
| Sorbitol | + |
| Inositol | - |
| Mannitol | + |
| Glycerol | + |
| Dextrin | + |
| Raffinose | + |
| (No gas formation from tested sugars) | |

The alkaline protease K-16 can be produced by culturing the strain KSM-16 in accordance, for example, with the Reference Example which will be described later, and isolating the enzyme from the resulting culture broth.

Although not particularly limited, an amount of enzymes in the solid enzyme preparation of the present invention may preferably be in the range of from 0.01 to 20 % by weight based on the total weight of the preparation.

If necessary, the solid enzyme preparation of the present invention may be mixed with a diluent (an extending agent, a filler and the like), a drying promotor, a buffer and the like, in order to maintain specific activity of the preparation at a constant level. As the drying promotor, calcium chloride, sodium sulfate and the like may be used. As to the extending agent or filler, a sulfate, a halide, a carbonate, a phosphate, a silicate, boric acid and a borate may be used. Specific examples of these salts include: sulfates such as sodium sulfate, potassium sulfate, calcium sulfate, magnesium sulfate, zinc sulfate, ferrous sulfate, sodium thiosulfate and aluminum sulfate; halides such as sodium chloride, potassium chloride, calcium chloride, magnesium chloride and potassium bromide; carbonates such as sodium carbonate, sodium hydrogencarbonate, potassium carbonate, calcium carbonate and magnesium carbonate; phosphates such as sodium phosphate, sodium hydrogenphosphate, sodium dihydrogenphosphate, potassium phosphate, potassium hydrogenphosphate, potassium dihydrogenphosphate and sodium pyrophosphate; silicates such as sodium silicate, sodium metasilicate, potassium silicate and calcium silicate; and boric acid and its salt such as borax and potassium borate. These additives may be used alone or as a mixture of two or more of them. In addition to these additives, a coloring agent, a stabilizer and the like generally used in the field of granulation or enzyme preparation may also be used, as well as a perfume, deodorant, an antistatic agent and the like.

The solid enzyme preparation of the present invention may be produced, for example, by solidifying an aqueous enzyme solution containing a disaccharide by means of spray drying in the usual way. In this instance, it is preferred that all components to be contained in the solid enzyme preparation are added to the aqueous enzyme solution containing the disaccharide.

In the practice of the process of the present invention, the aforementioned aqueous enzyme solution may be dried in the usual way using a spray dryer. Spray dryers are generally divided into a nozzle type and a disk type. These types may be selected according to the desired particle size of the solid enzyme preparation. That is, the nozzle type is preferable for the production of small particle size preparations, while the disk type is preferable for the production of large particle size preparations. In this way, any of powder, fine particle and granular preparation can be obtained.

Hot air drying may be carried out at a temperature of preferably from 100 to 200°C, more preferably from 130 to 170°C, with an exhaust air temperature (which corresponds to the temperature of the aqueous enzyme solution) preferably being in the range of from 50 to 100°C, more preferably from 60 to 100°C.

According to the present invention, the enzyme is hardly deactivated during spray drying treatment and, thereby, a solid enzyme preparation having high specific activity can be obtained. In addition, the solid enzyme preparation obtained in the process of the present invention has excellent thermal stability and it is not deactivated by heat treatment even after a prolonged period of treating time. The solid enzyme preparation of the present invention is also excellent in resistance to mechanical pressure.

Examples of the present invention are given below by way of illustration and not by way of limitation. Unless otherwise indicated, all percents except for activities of enzymes, are by weight.

## REFERENTIAL EXAMPLE 1

Isolation of protease-producing bacteria:

(1) About 1 g of soil sample was suspended in 10 ml of a sterilized physiological saline and the suspension was heat-treated at 80°C for 20 minutes. After the heat treatment, a 0.1 ml portion of the resulting supernatant was inoculated on a keratin halo agar medium and subjected to static culturing at 30°C for 48 hours. Composition of the keratin halo agar medium is as follows.

| Glucose | 1% |
| Yeast extracts | 0.2% |
| Animal hair keratin | 1% |
| Carboxymethyl cellulose | 1% |
| $KH_2PO_4$ | 0.1% |
| $MgSO_4 \cdot 7H_2O$ | 0.02% |
| Agar | 1.5% |

(2) The final pH of the above medium composition was adjusted to 10.5 by adding 1 % of a separately sterilized 10 % sodium carbonate solution, and the resulting medium was solidified in plates.

After the static culturing, colonies showing a halo (clear zone) around them were isolated and cultured again two to three times using the plate medium of the same composition to obtain purified protease-producing strains.

(3) The thus obtained strains were inoculated into a liquid medium having the following composition.

| Glucose | 2.0% |
| Polypeptone S | 1.0% |
| Yeast extracts | 0.05% |
| $KH_2PO_4$ | 0.1% |
| $MgSO_4 \cdot 7H_2O$ | 0.02% |
| Sodium carbonate (separate sterilization) | 1.0% |
| pH | 10.5 |

After culturing aerobically at 30°C for 48 hours on a shaker, the resulting culture broth was centrifuged at 3,000 rpm for 10 minutes to remove cells, and the resulting culture supernatant was used as an enzyme solution.

(4) Crude enzyme samples were prepared from the resulting enzyme solutions by means of freeze-drying and then examined for their storage stabilities at 40°C in a commercially available liquid detergent.

As the results, Bacillus sp. KSM-K16 was obtained as a strain capable of producing a protease having the highest stability.

## REFERENTIAL EXAMPLE 2

Culturing of cells and purification of Alkaline Protease K-16

(1) The alkalophilic bacterium, Bacillus sp. KSM-K16, obtained in Referential Example 1 was inoculated into the following liquid medium (3.0 liters) and cultured aerobically at 30°C for 48 hours on a shaker to produce the alkaline protease K-16.

| | |
|---|---|
| Glucose | 2.0% |
| Fish meat extracts | 1.0% |
| Soybean powder | 1.0% |
| $MgSO_4$ | 0.02% |
| $KH_2PO_4$ | 0.1% |
| pH | 10.0 |

(2) After the culturing, 3 liters of the thus obtained culture broth was centrifuged at 10,000 rpm for 5 minutes to remove cells, and the resulting culture supernatant was freeze-dried. A 2 g portion of the freeze-dried powder was dissolved in 10 ml of ion exchanged water to obtain a crude enzyme solution. The solution was dialyzed overnight against a 10 mM Tris-HCl buffer (supplemented with 2 mM $Ca^{2+}$, pH 7.5) using a dialysis membrane to obtain 26 ml of a dialyzed solution (activity: 3.15 P.U./ml; specific activity: 1.97 P.U./mg protein). Next, the thus dialyzed solution was applied to a column packed with CM-52 cellulose which has been equilibrated with a 10 mM Tris-HCl buffer (supplemented with 2 mM $Ca^{2+}$, pH 7.5). After washing the column with the same buffer, the alkaline protease K-16 was eluted with the same buffer containing 0 to 0.15 M sodium chloride and active fractions were pooled. The thus pooled fraction (15 ml) showed an activity of 1.12 P.U./ml and a specific activity of 5.75 P.U./mg protein. The pooled sample was dialyzed overnight against 50 mM Tris-HCl buffer (supplemented with 10 mM $Ca^{2+}$ and 0.2 M sodium chloride, pH 8.0), concentrated by ultrafiltration (cut off-molecular weight of ultrafiltration membrane: 5,000; manufactured by Amicon Corp.) and then applied to gel filtration chromatography using Sephadex G-50 (trade mark, manufactured by Pharmacia Inc.) which has been equilibrated with 50 mM Tris-HCl buffer (supplemented with 10 mM $Ca^{2+}$ and 0.2 M sodium chloride, pH 8.0). Elution was carried out using the same buffer to pool 11.5 ml of active fractions having an activity of 0.9 P.U./ml and a specific activity of 6.03 P.U./mg protein. Thereafter, the thus pooled sample was dialyzed overnight against ion-exchange treated water to obtain a solution having an activity of 0.56 P.U./ml and a specific activity of 5.60 P.U./mg protein.

The thus purified alkaline protease K-16 has the following enzymatic properties. In this instance, enzyme activity was measured in the following manner.

A 1 ml portion of 50 mM boric acid-NaOH buffer (pH 10) containing 1% casein was mixed with 0.1 ml of enzyme solution, and the mixture was incubated at 40°C for 10 minutes. The reaction was stopped by adding 2 ml of a solution containing 0.123 M trichloroacetic acid, 0.246 M sodium acetate and 0.369 M acetic acid. After allowing to stand at 30°C for 20 minutes, the resulting mixture was filtered through a filter paper (manufactured by Whatman; No. 2), and the filtrate was checked for its contents of proteolytic products in accordance with the modified Folin-Lowry method. One unit of activity (1 P.U.) was defined as the amount of enzyme which produces 1 mmole of tyrosine per minute.

(1) Action:

This enzyme reacts with various protein substrates under highly alkaline conditions.

(2) Substrate specificity:

Substrate specificity of the alkaline protease K-16 was examined by measuring its activity to hydrolyze casein, urea modified hemoglobin, animal hair keratin and elastin, and the results were compared with those of other proteases on the market. Each substrate was dissolved in 50 mM boric acid-NaOH buffer (pH 10.0) to a final concentration of 1% (provided that in the case of urea modified hemoglobin was to 2.2%). Each enzyme solution (in the case of elastin: $3.5 \times 10^{-4}$ P.U.; in the cases of other substrates: $0.5 \times 10^{-4}$ P.U.) was added to the thus prepared substrate solution and incubated at 40°C for 10 minutes. Relative activities of these enzymes to hydrolyze each substrate when the activity of the alkaline protease K-16 is regarded as 100 are shown in Table 2.

TABLE 2

| Enzymes | Substrates | | | |
|---|---|---|---|---|
| | Casein | Urea modified Hemoglobin | Animal Hair Keratin | Elastin |
| K-16 | 100 | 100 | 100 | 100 |
| Commercial Enzyme A | 100 | 108 | 100 | 77 |
| Commercial Enzyme B | 100 | 100 | 103 | 76 |

As is evident from the results, the alkaline protease K-16 hydrolyzes both water soluble and water insoluble proteinous materials, especially elastin, when compared with the commercial proteases A and B which are known as typical enzymes for detergent use.

(3) Optimum pH:

Each of various buffer solutions (50 mM) containing casein at a final concentration of 0.91% was mixed with $5.2 \times 10^{-5}$ P.U. of the alkaline protease K-16, and the mixture was incubated at 40°C for 10 minutes to measure its protease activity. As the results, the optimum pH of the alkaline protease K-16 was found to be 11.0 - 12.3. Buffers used in this experiment and their pH ranges are as follows.

| Acetate buffer | pH 3.9 - 5.7 |
|---|---|
| Phosphate buffer | pH 6.6 - 8.3 |
| Carbonate buffer | pH 9.2 - 10.9 |
| Phosphate-NaOH buffer | pH 10.9 - 12.7 |
| KCl-NaOH buffer | pH 10.9 - 12.6 |

(4) pH Stability:

Each of the just described five buffers (20 mM) was mixed with $7.9 \times 10^{-3}$ P.U. of the alkaline protease K-16, and the mixture was maintained at 25°C for 48 hours. Thereafter, the thus treated solution was diluted with 50 mM boric acid-NaOH buffer (pH 10.0) by a factor of 40 to measure residual activity. As the results, stable pH range of the alkaline protease K-16 was found to be pH 5.5 to 12.0 in the absence of $Ca^{2+}$, while the range was 5.0 to 12.0 in the presence of 2 mM $Ca^{2+}$.

(5) Optimum temperature:

A 50 mM boric acid-NaOH buffer (pH 10.0) containing 0.91% of casein as a substrate was mixed with $3.1 \times 10^{-5}$ P.U. of the alkaline protease K-16, and the mixture was incubated for 10 minutes at a predetermined temperature. In this manner, relative activity at each reaction temperature was calculated based on the activity at 40°C as 100%. As the results, the optimum temperature of the alkaline protease K-16 was found to be 55°C in the absence of $Ca^{2+}$ or 70°C in the presence of 5 mM $Ca^{2+}$.

(6) Thermal stability:

A $1.6 \times 10^{-3}$ P.U. portion of the alkaline protease K-16 was dissolved in 20 mM boric acid-NaOH buffer (pH 9.5), and the solution was treated for 10 minutes at a predetermined temperature. After cooling in an ice bath, the thus heat-treated sample was diluted with 50 mM boric acid-NaOH buffer (pH 10.0) by a factor of 5. Thereafter remained activity in the sample was measured using 0.91% casein as a substrate. In this manner, relative activity at each treating temperature was calculated based on the activity before the treatment as 100%. Under the above heat-treating conditions, 90% or more activity was maintained up to 50°C in the absence of $Ca^{2+}$ and to 60°C in the presence of 5 mM $Ca^{2+}$.

(7) Molecular weight:

Molecular weight of the alkaline protease K-16 was determined to be 28,000 ± 1,000 from sodium

EP 0 501 375 A1

dodecyl sulfate (SDS)-polyacrylamide gel electrophoresis. As molecular weight markers, a marker kit for low molecular weight use (Bio-Rad Laboratories, Inc.) was used which contained phosphorylase b (molecular weight, 97,400), bovine serum albumin (molecular weight, 66,200), ovalbumin (molecular weight, 42,700), carbonic anhydrase (molecular weight, 31,000), soybean trypsin inhibitor (molecular weight, 21,500) and lysozyme (molecular weight, 14,400).

(8) Isoelectric point:

Isoelectric point of the alkaline protease K-16 was found to be 10.5 or higher when examined by means of isoelectric focusing using Servalite 9-11 as amphoteric carrier for column use.

(9) Effects of metal ions:

Effects of various metal ions on the activity of the alkaline protease K-16 were examined. A 20 mM boric acid-NaOH buffer (pH 9.5) solution containing 1 mM of a metal salt was mixed with $3.9 \times 10^{-3}$ P.U. of the alkaline protease K-16, and the mixture was maintained at 30°C for 20 minutes. Thereafter, the thus treated mixture was diluted with 50 mM boric acid-NaOH buffer (pH 10.0) by a factor of 5 to measure residual activity which was then calculated as relative value based on the activity measured in the same manner in the absence of metal salts. As shown in Table 3, the activity of the alkaline protease K-16 is inhibited by $Hg^{2+}$ and $Cu^{2+}$. Also, as is evident from the results described in the foregoing items (5) and (6), thermal stability of this enzyme is improved in the presence of $Ca^{2+}$

TABLE 3

| Metal salt (1 mM) | Residual Activity (%) |
|---|---|
| No addition | 100 |
| $ZnCl_2$ | 106 |
| $AgNO_3$ | 86 |
| $CaCl_2$ | 103 |
| $NiCl_2$ | 103 |
| $CoCl_2$ | 103 |
| $PbCl_2$ | 100 |
| $HgCl_2$ | 46 |
| $CuSO_4$ | 73 |

(10) Effects of inhibitors:

Effects of generally used enzyme inhibitors on the activity of the alkaline protease K-16 were examined. A 10 mM phosphate buffer (pH 7.0) solution containing a predetermined amount of each of the inhibitors was mixed with $7.9 \times 10^{-3}$ P.U. of the alkaline protease K-16, and the mixture was maintained at 30°C for 20 minutes. Thereafter, the thus treated mixture was diluted with ion exchanged water by a factor of 20 to measure residual activity which was then calculated as relative value based on the activity measured in the same manner in the absence of inhibitors. As shown in Table 4, the activity of the alkaline protease K-16 was inhibited by diisopropyl fluorophosphate (DFP), phenylmethane sulfonyl fluoride (PMSF) and chymostatin. Since these compounds are known as serine protease inhibitors, it is evident that the alkaline protease K-16 is an enzyme which has a serine residue in its active center.

8

## TABLE 4

| Inhibitors | Concentration | Residual Activity (%) |
|---|---|---|
| No addition | – | 100 |
| EDTA *1 | 5 mM | 107 |
| PCMB *2 | 1 mM | 100 |
| DFP *3 | 1 mM | 3.8 |
| PMSF *4 | 1 mM | 1.5 |
| Antipain | 0.01% | 108 |
| Chymostatin | 0.01% | 34 |

Notes:   *1: EDTA; ethylenediaminetetraacetic acid

*2: PCMB; p-chloromercuribenzoic acid

*3: DFP; diisopropyl fluorophosphate

*4; PMSF; phenylmethanesulfonyl fluoride

(11) Effects of surface active agents:

A $6.6 \times 10^{-2}$ P.U. portion of an enzyme solution was added to 5 ml of 0.1 M Tris-HCl buffer (pH 9.0, contains 10% ethanol) in which a predetermined amount of a surface active agent has been dissolved, and the mixture was maintained at 40°C for 4 hours. Thereafter, the thus treated mixture was diluted with 50 mM boric acid-NaOH buffer (pH 10.0) by a factor of 20 to measure residual activity which was then calculated as relative value based on the activity measured at 0 hour of the treatment. The results are shown in Table 5. As is evident from the results, the alkaline protease K-16 is highly stable in the presence of various surface active agents even at their high concentrations (1 to 10%).

9

<u>TABLE 5</u>

| Surface active agents (Conc.) | K-16 (%) | Commercial Enzyme | |
|---|---|---|---|
| | | B (%) | C (%) |
| Straight chain sodium alkylbenzene sulfonate *1 (1%) | 65 | 48 | 46 |
| Sodium polyoxyethylene alkyl sulfate *2 (1%) | 100 | 97 | 52 |
| Sodium dodecyl sulfate *3 (10%) | 58 | 0 | 48 |
| Sodium a-olefin sulfonate *4 (1%) | 100 | 82 | 61 |
| Sodium alkane sulfonate *5 (10%) | 81 | 23 | 72 |
| α-sulfo-fatty acid ester *6 (1%) | 100 | 86 | 75 |
| Softanol 70H (Nippon Shokubai Kagaku Kogyo Co., Ltd.) *7 (1%) | 100 | 84 | 90 |

Notes:   *1: R–⬡–$SO_3Na$ (R: $C_{10}$ – $C_{14}$)

*2: $R-CH_2O(C_2H_4O)_nSO_3Na$ (R: $C_9$ – $C_{17}$; n = 1 – 5)

*3: $R-CH_2OSO_3Na$ (R: $C_9$ – $C_{17}$, but mainly $C_{12}$)

*4: $R-CH=CH(CH_2)_nSO_3Na$ (R: $C_7$ – $C_{15}$; n = 0.1 – 5)

*5: R–CH–R′
      |                    (R + R′: $C_{13}$ – $C_{18}$)
     $SO_3Na$

*6: R–CH–COCR′
      |                    (R: $C_{10}$ – $C_{16}$; R′: $C_1$ – $C_6$)
     $SO_3Na$

*7: $R-CH_2O(C_2H_4O)_nH$ (R: $C_8$ – $C_{17}$; n = 5 – 15)

EXAMPLE 1

(1) An aqueous solution containing 5% of the crude alkaline protease K-16 preparation obtained in Reference Example 1, 0.2% of calcium chloride and 2.5% of sodium sulfate was used as an enzyme solution in this experiment. Sample Nos. 1 to 4 were prepared by adding 0.5 to 5.0% of lactose to the enzyme solution.

(2) The thus prepared Samples Nos. 1 to 4 and a control sample (no addition of lactose) were subjected to spray drying using an atomizer type spray dryer (hot air temperature: 150°C; exhaust air temperature:

60°C) to obtain solid enzyme preparations in the form of granule.

(3) Enzyme activities before and after the spray drying were measured to calculate activity yield of the enzyme. The results shown in Table 6.

TABLE 6

| 5% Enzyme Solution (Lactose Concentration) | Enzyme Activity Yield (%) |
|---|---|
| Control | 83 |
| Sample 1 (0.5 %) | 90 |
| Sample 2 (1.0 %) | 96 |
| Sample 3 (2.5 %) | 96 |
| Sample 4 (5.0 %) | 95 |

As is evident from the results shown in Table 6, the alkaline protease K-16 was not deactivated by spray drying when lactose was added to the enzyme solution.

EXAMPLE 2

Granular solid enzyme preparations were prepared and their enzyme activity yields were measured by repeating the procedure of Example 1 except that the exhaust air temperature was varied within the range of from 60 to 70°C. The results are shown in Table 7.

TABLE 7

| 5 % Enzyme solution (Lactose Concentration) | Enzyme Activity Yield | | |
|---|---|---|---|
| | 60°C (%) | 65°C (%) | 70°C (%) |
| Control | 83 | 79 | 70 |
| Sample 1 (0.5%) | 96 | 99 | 96 |
| Sample 2 (1.0%) | 94 | 95 | 95 |

As is evident from the results shown in Table 7, the alkaline protease K-16 was not deactivated by spray drying, even under severe drying conditions, when lactose was added to the enzyme solution.

EXAMPLE 3

The granular solid enzyme preparations obtained in Example 1 were maintained for 1 hour under a heating condition of 90°C and residual activities in the resulting preparations were measured to evaluate their thermal stabilities. The results are shown in Table 8.

TABLE 8

| 5% Enzyme solution (Lactose Concentration) | Residual Activity (%) |
|---|---|
| Control | 84 |
| Sample 1 (0.5%) | 95 |
| Sample 2 (1.0%) | 96 |
| Sample 3 (2.5%) | 96 |
| Sample 4 (5.0%) | 97 |

As is evident from the results shown in Table 8, the enzyme preparation obtained by the process of the present invention has excellent thermal stability in its solid form.

While the invention has been described in detail and with reference to specific examples thereof, it will be apparent to one skilled in the art that various changes and modifications can be made therein without departing from the spirit and scope thereof.

**Claims**

1. A solid enzyme preparation comprising an enzyme and a disaccharide.

2. A solid enzyme preparation of claim 1, wherein said disaccharide is a compound selected from maltose type disaccharides and trehalose type disaccharides.

3. A solid enzyme preparation of claim 2, wherein said maltose type disaccharide is lactose.

4. A solid enzyme preparation of claim 1 or 2, wherein said disaccharide is present in an amount of from 1 to 100 % by weight based on the weight of said enzyme.

5. A solid enzyme preparation of claim 1 or 2, wherein said disaccharide is present in an amount of from 10 to 100% by weight based on the weight of said enzyme.

6. A solid enzyme preparation of claim 3, wherein lactose is present in an amount of from 1 to 100% by weight based on the weight of said enzyme.

7. A solid enzyme preparation of claim 3, wherein lactose is present in an amount of from 10 to 100% by weight based on the weight of said enzyme.

8. A solid enzyme preparation of claim 1, wherein said enzyme is an enzyme selected from the group consisting of a lipase, a cellulase, a protease, an amylase, an esterase and a dextranase.

9. A solid enzyme preparation of claim 8, wherein said enzyme is a cellulase, a protease or a mixture of a cellulase and a protease.

10. A solid enzyme preparation of claim 8 or 9, wherein said enzyme is an alkaline protease K-16.

11. A solid enzyme preparation of any of claims 1, 8 and 9, wherein said enzyme is present in an amount of from 0.01 to 20 % by weight in said solid enzyme preparation.

12. A solid enzyme preparation of claim 10, wherein said alkaline protease K-16 is present in an amount of from 0.01 to 20% by weight in said solid enzyme preparation.

13. A process for producing a solid enzyme preparation which comprises solidifying an aqueous enzyme solution containing a disaccharide by spray drying.

14. A process of claim 13, wherein said disaccharide is a compound selected from maltose type disaccharides and trehalose type disaccharides.

15. A process of claim 14, wherein said maltose type disaccharide is lactose.

16. A process of any of claim 13 or 14, wherein said disaccharide is present in an amount of from 1 to 100 % by weight based on the weight of said enzyme.

17. A process of claim 16, wherein said disaccharide is present in an amount of from 10 to 100 % by weight based on the weight of said enzyme in said aqueous enzyme solution.

18. A process of claim 15, wherein said lactose is present in an amount of from 1 to 100% by weight based on the weight of said enzyme.

19. A process of claim 18, wherein said lactose is present in an amount of from 10 to 100% by weight based on the weight of said enzyme.

20. A process of claim 18, wherein said spray drying is carried out at a temperature of 50°C or more.

21. A process of claim 18, wherein said spray drying is carried out using a hot air of 100 to 200°C.

22. A process of claim 18, wherein said spray drying is carried out using a hot air of 130 to 170°C.

23. A process of claim 18, wherein said enzyme is an enzyme selected from the group consisting of a lipase, a cellulase, a protease, an amylase, an esterase and an dextranase.

24. A process of claim 22, wherein said enzyme is a cellulase, a protease or a mixture of a cellulase and a protease.

25. A process of claim 18 or 23, wherein said enzyme is an alkaline protease K-16.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | NETHERLANDS MILK AND DAIRY JOURNAL. vol. 23, 1969, WAGENINGEN NL pages 46 - 54; M.J. VAN DE BEEK ET AL.: 'Preservation of the enzymatic activity of rennin during spray drying and during storage, and the effect of sugars and certain other additives' * page 46, paragraph 1 -last paragraph * * page 47, paragraph 5 * * page 48, paragraph 3 - page 50, paragraph 1; tables 1-4 * * page 52, paragraph 5 - page 53, paragraph 1 * | 1-9,11, 13-24 | C12N9/96 |
| X | CHEMICAL ABSTRACTS, vol. 80, no. 11, 27 May 1974, Columbus, Ohio, US; abstract no. 119231T, MASUDA, KAZUO: 'Stable protease powder' page 309 ; column R ; * abstract * & & JP-73 34912 (HAYA-SHIBARA CO., LTD.) 24-10-73 | 1,2,4,5, 8,9,11, 13,14, 16,17, 20,23,24 | |
| X | FR-A-746 391 (KALLE & C AKTIENGESELLSCHAFT) * the whole document * | 1-4,6,8, 11, 13-16, 18,23 | TECHNICAL FIELDS SEARCHED (Int. Cl.5) C12N |
| X | PATENT ABSTRACTS OF JAPAN vol. 10, no. 120 (C-343)(2177) 6 May 1986 & JP-A-60 244 288 ( OOKURA SEIYAKU K.K. ) 4 December 1985 * abstract * | 1-3,8,9, 13-15, 21,23,24 | |

The present search report has been drawn up for all claims

| Place of search | Date of completion of the search | Examiner |
|---|---|---|
| THE HAGUE | 07 MAY 1992 | MONTERO LOPEZ B. |

EPO FORM 1503 03.82 (P0401)